# EUROPEAN PATENT APPLICATION

(11) **EP 1 400 535 A1**
(43) Date of publication of application: **24.03.2004**
(21) Application number: 02020334.5
(22) Date of filing: 11.09.2002
(51) Int. Cl.: C07K 16/28, A61K 39/395

(54) **Therapeutic anti-BGP (C-CAM1) antibodies and uses thereof**

(71) Applicant: The General Hospital Corporation doing business as Massachusetts General Hospital, Boston, MA 02114 (US); BRIGHAM AND WOMEN'S HOSPITAL, INC., Boston, MA 02115 (US)
(72) Inventor: Blumberg, Richard S., Chestnut Hill, MA 02467 (US); Bhan, Atul K., West Newton, MA 02465 (US)
(74) Representative: Knauthe

(57) **Abstract**

Provided are specific antibodies which are capable of modulating T cell activity and uses thereof. In particular, antibodies and binding fragments thereof are described, which react with a specific domain of biliary glycoprotein (BGP), also known as CD66a, CEACAM1 and C-CAM1, and which are capable of suppressing the cytolytic activity of intestinal intraepithelial lymphocytes (iIELs). Furthermore, compositions comprising said antibodies are provided and methods of modulating immune cell proliferation, and treating immune response related diseases.

## Description

### Field of the invention

The present invention relates to antibodies which are capable of modulating T cell activity and uses thereof. In particular, the antibodies of the invention and binding molecules derived thereof react with a specific domain of biliary glycoprotein (BGP), also known as CD66a, CEACAM1 and C-CAM1, and are capable of modulating T cell activity. Furthermore, the present invention relates to compositions comprising said antibodies and to methods of modulating immune cell proliferation, and treating immune response related diseases.

Several documents are cited throughout the text of this specification. Each of the documents cited herein (including any manufacturer's specifications, instructions, etc.) are hereby incorporated herein by reference; however, there is no admission that any document cited is indeed prior art as to the present invention.

### Background art

T-cell activation is a serial process involving multiple signaling pathways and sequential changes in gene expression resulting in differentiation of T-cells into distinct subpopulations, i.e. Th1 and Th2, which are distinguishable by their pattern of cytokine production and characterize the mode of cellular immune response. The T-cell response is initiated by the interaction of the antigen-specific T-cell receptor (TCR) with peptide presented by major histocompatibility complex (MHC) molecules on the surface of antigen presenting cells (APCs). Additional signals are provided by a network of receptor-ligand interactions mediated by a number of membrane proteins such as CD28/CTLA4 and B7, CD40/CD40L, LFA-1 and ICAM-1 (Lenschow, Science 257 (1992), 789-792; Linsley, Annu. Rev. Immunol. 11 (1993), 191-212; Xu, Immunity 1 (1994), 423-431; Bachmann, Immunity 7 (1997), 549-557; Schwartz, Cell 71 (1992), 1065-1068) collectively called costimulatory signals (Perez, Immunity 6 (1997), 411). These membrane proteins can alter T-cell activation in distinct ways (Bachmann, Immunity 7 (1997), 549-557) and regulate the immune response by the integration of positive and negative signals provided by these molecules (Bluestone, Immunity 2 (1995), 555-559; Perez, Immunity 6 (1997), 411). Many of the agents which are effective in modulating the cellular immune response either interfere with the T-cell receptor (Cosimi, Transplantation 32 (1981), 535-539) block costimulatory signaling (Larsen, Nature 381 (1996), 434-438; Blazar J. Immuno. 157 (1996), 3250-3259; Kirk, Proc. Natl. Acad. Sci. USA 94 (1997), 8789-8794; Linsley, Science 257 (1992), 792-95; Turka, Proc. Natl. Acad. Sci. USA 89 (1992), 11102-11105) or inhibit intracellular activation signals downstream from these primary cell membrane triggers (Schreiber and Crabtree, Immunology Today 13 (1992), 136-42). Therapeutic prevention of T-cell activation in organ transplantation and autoimmune diseases presently relies on panimmunosupressive drugs interfering with downstream intracellular events. Specific modulation of the immune response remains a longstanding goal in immunological research.

Recently, it has been discovered that biliary glycoprotein (BGP, also known as CD66a, CEACAM1 and C-CAM1), a member of the carcinoembryonic antigen family (CEA), is an inhibitory receptor for activated T cells contained within the human intestinal epithelium; see WO99/52552 and Morales et al. J. Immunol. 163 (1999), 1363 - 1370. These studies suggest that, in a regional microenvironment that is predominantly CD28/CTLA4-CD80/CD86 negative, other receptor-ligand interactions may provide necessary down-regulatory signals to limit T cell activation and immunopathology. Furthermore, it has been shown that C-CAM1 is expressed on T cells in the periphery (peripheral blood, etc.) outside the gut and intestinal epithelium in particular. Therefore, C-CAM1 needs to be considered as a T cell activation antigen on T cells in general and not just limited to the gut. This therefore makes C-CAM1 relevant to a wide variety of immune-mediated processes.

In view of the need of therapeutic means for the treatment of diseases related to immune responses of the human body, the technical problem of the present invention is to provide means and methods for modulation of the immune response in a subject. The solution to said technical problem is achieved by providing the embodiments characterized in the claims, and described further below.

### Summary of the invention

The present invention is directed to antibodies or antigen-binding fragments and similar BGP binding molecules which are capable of modulating T cell activity and binding to an epitope of the N-domain of BGP (C-CAM1).

A particularly preferred embodiment is a monoclonal antibody or antigen-binding fragment thereof which demonstrates the immunological binding characteristics of monoclonal antibody 34B1 as produced by hybridoma 34B1, deposited with the American Type Culture Collection (ATCC) 10801 University Blvd. Manassas, VA 20110-2209 U.S.A. on September 4, 2002, and assigned Accession Number ..........; 26H7 as produced by hybridoma 26H7, deposited with the American Type Culture Collection (ATCC) on September 4, 2002, and assigned Accession Number ..........; or 5F4 as produced by hybridoma 5F4, deposited with the American Type Culture Collection (ATCC) on September 4, 2002, and assigned Accession Number ...........

In a preferred aspect, the antibody is a human, humanized, xenogeneic, or a chimeric human-murine antibody. Therapeutic compositions including the antibody or active fragments thereof, or agonists and cognate molecules, or alternately, antagonists of the same, and methods of use of such compositions in the prevention, diagnosis or treatment of disease using these compositions are also included, wherein an effective amount of the composition is administered to a patient in need of such treatment.

The antigen-binding fragment of the monoclonal antibody can be a single chain Fv fragment, an F(ab') fragment, an F(ab) fragment, and an F(ab')₂ fragment, or any other antigen-binding fragment. In a specific embodiment, infra, the monoclonal antibody or fragment thereof is a murine IgG isotype antibody.

Naturally, the invention extends to the hybridoma that produces monoclonal antibody 34B1, 26H7 or 5F4, which hybridoma is deposited with the ATCC as indicated hereinbefore.

The present invention also relates to polynucleotides encoding at least a variable region of an immunoglobulin chain of the antibody of the invention. Preferably, said variable region comprises at least one complementarity determining region (CDR) of the V_{H} and/or V_{L} of the variable region of the antibody 34B1, 26H7 or 5F4 .

Accordingly, the present invention also encompasses vectors comprising said polynucleotides and host cells transformed therewith as well as their use for the production of an antibody capable of modulating T cell activity or a functional fragment or immunoglobulin chain(s) thereof.

The antibody, immunoglobulin chain(s), binding fragments thereof and ligands other than BGP binding to said antibody can be used in pharmaceutical and diagnostic compositions for modulating and detecting an immune response, respectively.

The use of the foregoing compositions in the preparation of medicament is preferred. In preferred embodiments, the medicament is useful in the treatment of conditions related to immune system function, including autoimmune or immune mediated disorders and diseases. Disorders would include autoimmune (e.g. inflammatory bowel disease, rheumatoid arthritis, psoriasis, multiple sclerosis), transplantation (e.g. kidney, heart) and cancer (e.g. colon, melanoma) wherein T cell activities would need to be increased or decreased where appropriate.

### Brief Description of the Drawings

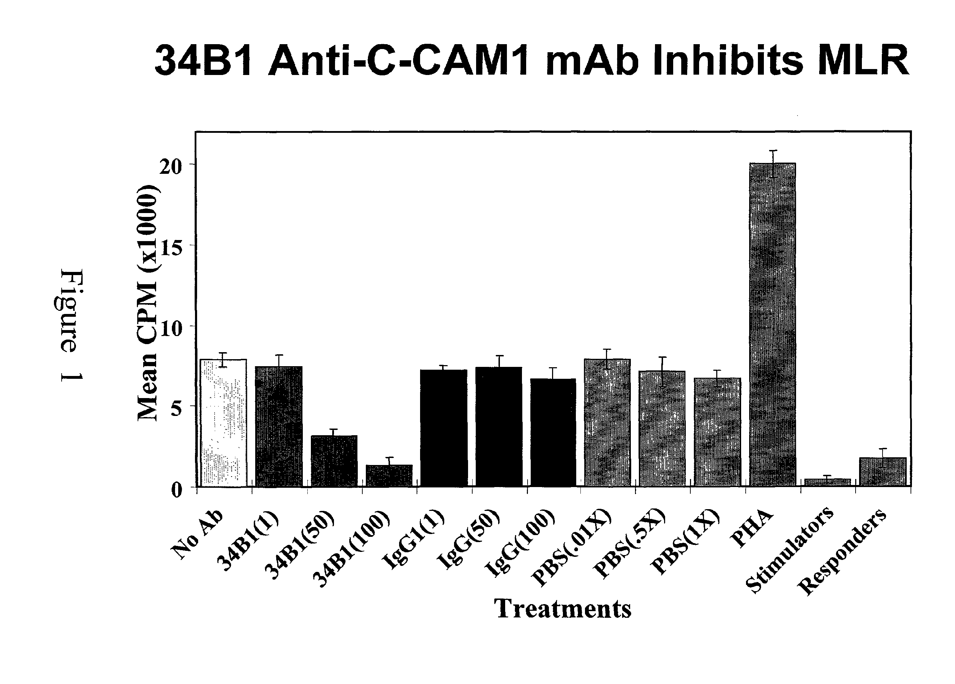

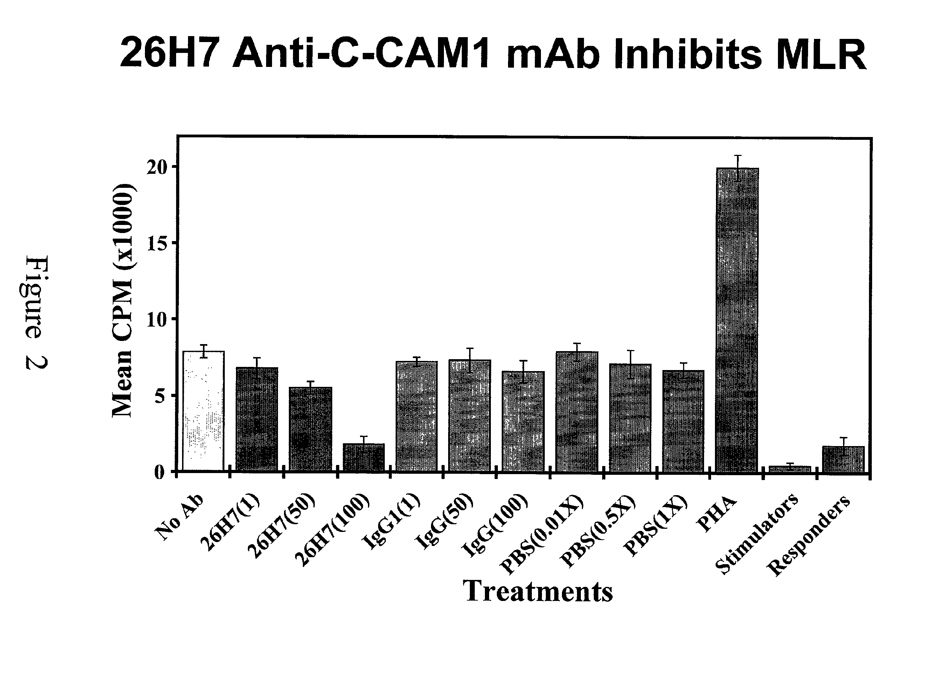

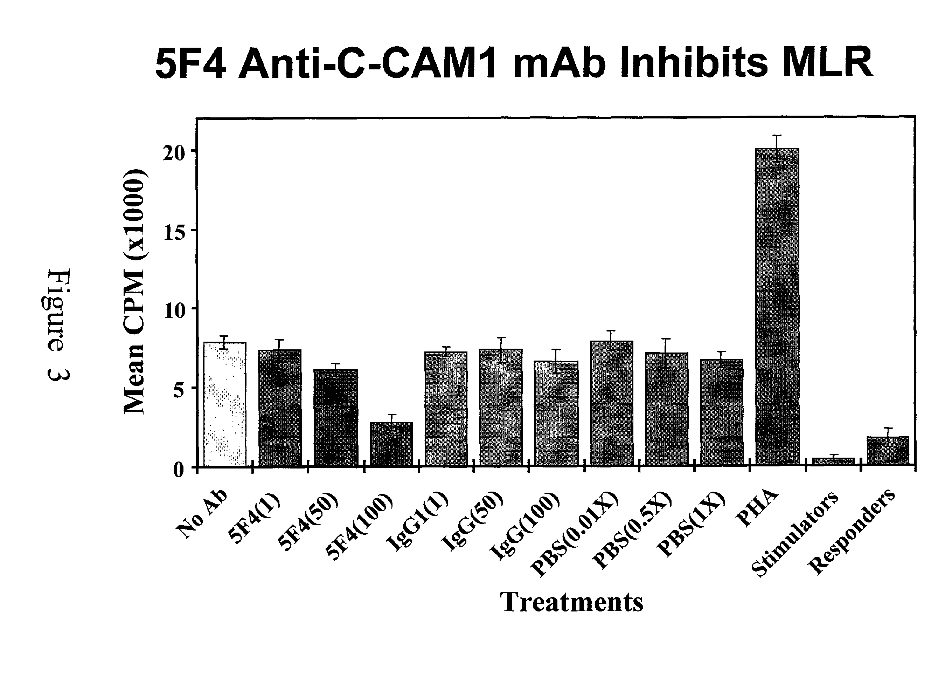

**Figures 1-3:** 34B1, 26H7 and 5F4 anti-C-CAM1 monoclonal antibodies inhibit Mixed Lymphocyte Reaction (MLR).

### Description of the Invention

The present invention relates to antibodies and binding molecules which are capable of modulating killer and other functional activities of T cells and which react with an epitope of the N-domain of BGP (C-CAM1).
In particular, the antibody or antigen-binding fragment thereof of the present invention demonstrate the immunological binding characteristics of monoclonal antibody 34B1 as produced by hybridoma 34B1, deposited with the American Type Culture Collection (ATCC) 10801 University Blvd. Manassas, VA 20110-2209 U.S.A. on September 4, 2002, and assigned Accession Number ..........; 26H7 as produced by hybridoma 26H7, deposited with the American Type Culture Collection (ATCC) on September 4, 2002, and assigned Accession Number ..........; or 5F4 as produced by hybridoma 5F4, deposited with the American Type Culture Collection (ATCC) on September 4, 2002, and assigned Accession Number ...........
Where present, the term "immunological binding characteristics," or other binding characteristics of an antibody with an antigen, in all of its grammatical forms, refers to the specificity, affinity, cross-reactivity, and other binding characteristics of an antibody.

Naturally, the invention extends to the hybridoma as well. Thus, the invention advantageously provides an indefinitely prolonged cell source of a monoclonal antibody of the invention: the hybridoma. Hence, the present invention also encompasses a method for producing anti-C-CAM1 antibodies comprising culturing the hybridoma cell of the invention and isolating antibodies from the culture or cell or media. The invention further relates to diagnostic assay methods and kits that comprise the monoclonal antibody of the invention and to therapeutic methods based thereon.
Evidence for a role of human biliary glycoprotein (BGP, C-CAM1) as an inhibitory molecule on activated iIELs has been provided in WO99/52552. Human BGP is a member of the CEA family of glycoproteins, part of the immunoglobulin supergene family, and encoded in a large cluster on chromosome 19 (Watt et al., Blood 84 (1994), 200-210, Daniel et al., Int. J. Cancer 55 (1993), 303-310; Teixeira et al., Blood 84 (1994), 211-219; Barnett et al., Mol. Cell. Biol. 13 (1993), 1273-1282). The CEA-cluster is highly related to the genetically linked, pregnancy specific gene cluster (Thompson et al., J. Clin. Lab. Anal. 5 (1991), 344-366; Obrink, Curr. Opin. Cell Biol. 9 (1997), 616-626). The CEA-subgroup of this family is serologically defined as CD66a (BGP or C-CAM1), CD66b (CGM6), CD66c (NCA), CD66d (CGM1) and CD66e (CEA). These structurally related glycoproteins consist of a highly homologous membrane distal amino terminal IgV-like N-domain and variable numbers of membrane distal IgC2-like domains in the case of BGP, NCA, CGM6 and CEA. In contrast to human CEA, CGM6 and NCA, which are linked to the membrane by a glycosyl phosphatidyl-inositol anchor, CGM1 and BGP are type 1 transmembrane glycoproteins. Both of the latter exist as isoforms containing short or long cytoplasmic tails.
BGP, and its mouse and rat homologues C-CAM (Rosenberg et al., Cancer Res. 53 (1993), 4938-4945; Lin et al., J. Biol. Chem. 264 (1989), 14408-14414; Obrink, BioEssays 13 (1991), 227-234,), have been regarded mainly as molecules which function in cell-cell adhesion that are expressed primarily by epithelial cells of the gastrointestinal tract and biliary tree, neutrophils and, more recently, B cells. BGP also serves as a receptor for mouse hepatitis virus (Williams et al., Proc. Natl. Acad. Sci. 88 (1991), 5533-5536) and for Opa proteins of Neisseria species of bacteria (Virji et al., Mol. Microbiol. 5 (1996), 941-950). It is of interest that ligation of BGP on epithelial cells may deliver a negative growth signal which may be decreased during tumor formation due to diminished expression of BGP (Rosenberg et al., 1993; Kunath et al., Oncogene, 11 (1995), 2375- 2382; Brumer et al., Oncogene, 11 (1995), 1649-1655).

Previous studies indicated that the expression of BGP on activated human iIELs and, potentially, other mucosal T lymphocytes is involved in the down-regulation of cytolytic function; see Morales et al., J. Immunol. 163 (1999), 1363-1370. Hence, compounds capable of modulating the cytolytic activity of intestinal intraepithelial lymphocytes (iIELs) could be envisaged, which exerts their modulatory function through BGP.
The present invention provides such molecules. In particular, monoclonal antibodies and antigen-binding fragments are provided, which bind to an epitope of the N-domain of BGP (C-CAM1) and which are capable of modulating T cell proliferation. While international application WO99/52552 generally teaches the use of anti-BGP monoclonal antibody for modulating T cell activity, the present application discloses three particular antibodies which distinguish themselves through different binding specificities for the different members of the CEA family and biological activities. Thus, each of the three monoclonal antibodies exemplified in the present application is unique with respect to its immunological and biological activities. Furthermore, the provision of the hybridomas producing monoclonal antibodies 26H7, 5F4 and 34B1, respectively, enables the person skilled in the art to design and produce functionally equivalent antibodies, for example by adapting the antigen binding side of any one of the three mentioned antibodies.

The monoclonal antibodies of the present invention may be distinguished from others by several means. First, the instant antibodies do react with BGP(C-CAM1). Second, they react with the N-domain of BGP but do not substantially react with the A1, B1 and A2 domain of BGP. Third, they are capable of inhibiting T cell proliferation in an allogenic mixed lymphocyte reaction and, fourth, suppressing the cytolytic activity of intestinal intraepithelial lymphocytes (iIELs).

Furthermore, antibodies 26H7 and 5F4 specifically recognize the N-domain of C-CAM1 but not the N-domain of CEA (C-CAM5), NCA (C-CAM6), CGM6 (C-CAM8) or CGM1 (C-CAM3); see also publication by Morales et al. in J. Immunol. 1999. Please be also referred to Exp. Cell Res. 252 (1999), 243, for the new nomenclature. There is no reason to believe that the 26H7 or 5F4 would recognize C-CAM 4 or 7 given their clear ability to distinguish C-CAM1 from the others, however, it may not be excluded. However, it should be recognized that the only C-CAM family member that is expressed by T cells (in the intestine or periphery by the work of the present inventors) is C-CAM1 (BGP). The 34B1 monoclonal antibody is different because it recognizes C-CAM1, 3, 5 and 6 but not C-CAM8 (CGM6). Therefore, antibodies 26H7 and 5F4 seem to be specific for C-CAM1. Noteworthy, in mouse studies with a mouse anti-mouse C-CAM1 monoclonal antibody, specific inhibition of T-helper 1 cytokine production has been observed by the inventors as well (e.g. gamma-interferon).
Monoclonal antibody 34B1 reacts with the native form of C-CAM1-FC protein and, to a lesser extent, with the denatured protein; see Watt et al., Blood 98, 1469-1479. In addition, by site directed mutagenesis, it could be shown the monoclonal antibody 5F4 in particular binds to a particular domain within the N-domain of C-CAM1 that is involved in homophilic interactions between the N-domains of different C-CAM1 molecules (Watt et al., Blood 98 (2001), 1469-1479) and by the recent crystal structure of mouse C-CAM1 (Tan et al. Embo J. 21 (2002), 2076-2086) within the region of the critical C-C loop involved not only in predicted homophilic binding but also to heterophilic interactions such as with microbes. One might therefore also predict that another utility of these antibodies or their by-products is as anti-infective agents. This is further supported by the recent publication in Nature Immunology (2002) 229-236 by Boulton and Gray-Owen showing that Neisserial binding to C-CAM1 arrests the activation of CD4+ T lymphoctes in humans.

In one embodiment, the antibody of the present invention is any one of monoclonal antibody 34B1 as produced by hybridoma 34B1, deposited with the American Type Culture Collection (ATCC) 10801 University Blvd. Manassas, VA 20110-2209 U.S.A. on September 4, 2002, and assigned Accession Number ..........; 26H7 as produced by hybridoma 26H7, deposited with the ATCC on September 4, 2002, and assigned Accession Number ..........; or 5F4 as produced by hybridoma 5F4, deposited with the ATCC on September 4, 2002, and assigned Accession Number ...........
Alternatively, the antibody of the present invention is a monoclonal antibody or antigen-binding fragment thereof, which competes for binding to C-CAM1 with at least one of the antibodies produced by the above-described hybridomas. Those antibodies may be murine as well, however, humanized, xenogeneic, or chimeric human-murine antibodies being preferred, in particular for therapeutic applications. An antigen-binding fragment of the antibody can be, for example, a single chain Fv fragment, an F(ab') fragment, an F(ab) fragment, and an F (ab')₂ fragment.

In accordance with the above, the present invention also relates to a hybridoma that produces any one of the above described monoclonal antibodies. Preferably, said hybridoma is selected from the group consisting of hybridoma 34B1, deposited with the American Type Culture Collection (ATCC) 10801 University Blvd. Manassas, VA 20110-2209 U.S.A. on September 4, 2002, and assigned Accession Number ..........; hybridoma 26H7, deposited with the ATCC on September 4, 2002, and assigned Accession Number .......... ; or hybridoma 5F4, deposited with the ATCC on September 4, 2002, and assigned Accession Number ..........;

Some of the mentioned embodiments are further explained below.

Thus, for some applications only the variable regions of the antibodies are required, which can be obtained by treating the monoclonal antibody isolated from the hybridoma with suitable reagents so as to generate Fab', Fab, or F(ab")₂ portions. Such fragments are sufficient for use, for example, in immunodiagnostic procedures involving coupling the immunospecific portions of immunoglobulins to detecting reagents such as radioisotopes.

As an alternative to obtaining immunoglobulins directly from the culture of hybridomas, the immortalized hybridoma cells can be used as a source of rearranged heavy chain and light chain loci for subsequent expression and/or genetic manipulation. Rearranged antibody genes can be reverse transcribed from appropriate mRNAs to produce cDNA. If desired, the heavy chain constant region can be exchanged for that of a different isotype or eliminated altogether. The variable regions can be linked to encode single chain Fv regions. Multiple Fv regions can be linked to confer binding ability to more than one target or chimeric heavy and light chain combinations can be employed. Once the genetic material is available, design of analogs as described above which retain both their ability to bind the desired target is straightforward. Methods for the cloning of antibody variable regions and generation of recombinant antibodies are known to the person skilled in the art and are described, for example, Gilliland et al., Tissue Antigens 47 (1996), 1-20; Doenecke et al., Leukemia 11 (1997), 1787-1792.

Once the appropriate genetic material is obtained and, if desired, modified to encode an analog, the coding sequences, including those that encode, at a minimum, the variable regions of the heavy and light chain, can be inserted into expression systems contained on vectors which can be transfected into standard recombinant host cells. A variety of such host cells may be used; for efficient processing, however, mammalian cells are preferred. Typical mammalian cell lines useful for this purpose include CHO cells, 293 cells, or NSO cells. The production of the antibody or analog is then undertaken by culturing the modified recombinant host under culture conditions appropriate for the growth of the host cells and the expression of the coding sequences. The antibodies are then recovered by isolating them from the culture. The expression systems are preferably designed to include signal peptides so that the resulting antibodies are secreted into the medium; however, intracellular production is also possible.

In accordance with the above, the present invention also relates to a polynucleotide encoding at least a variable region of an immunoglobulin chain of the antibody described above. Typically, said variable region encoded by the polynucleotide comprises at least one complementarity determining region (CDR) of the VH and/or VL of the variable region of the antibody produced by any one of the above described hybridomas. The person skilled in the art knows that each variable domain (the heavy chain VH and light chain VL) of an antibody comprises three hypervariable regions, sometimes called complementarity determining regions or "CDRs" flanked by four relatively conserved framework regions or "FRs". The CDRs contained in the variable regions of the antibody of the invention can be determined, e.g., according to Kabat, Sequences of Proteins of Immunological Interest (U.S. Department of Health and Human Services, third edition, 1983, fourth edition, 1987, fifth edition 1990). The person skilled in the art will readily appreciate that the variable domain of the antibody having the above-described variable domain can be used for the construction of other polypeptides or antibodies of desired specificity and biological function. Thus, the present invention also encompasses polypeptides and antibodies comprising at least one CDR of the above-described variable domain and which advantageously have substantially the same or similar binding properties as the antibody described in the appended examples. The person skilled in the art will readily appreciate that using the variable domains or CDRs described herein antibodies can be constructed according to methods known in the art, e.g., as described in EP-A1 0 451 216 and EP-A1 0 549 581. Furthermore, the person skilled in the art knows that binding affinity may be enhanced by making amino acid substitutions within the CDRs or within the hypervariable loops (Chothia and Lesk, J. Mol. Biol. 196 (1987), 901-917) which partially overlap with the CDRs as defined by Kabat. Thus, the present invention also relates to antibodies wherein one or more of the mentioned CDRs comprise one or more, preferably not more than two amino acid substitutions. Preferably, the antibody of the invention comprises in one or both of its immunoglobulin chains two or all three CDRs of the variable regions of antibodies 34B1, 26H7 or 5F4.

The polynucleotide of the invention encoding the above described antibody may be, e.g., DNA, cDNA, RNA or synthetically produced DNA or RNA or a recombinantly produced chimeric nucleic acid molecule comprising any of those polynucleotides either alone or in combination. Preferably said polynucleotide is part of a vector. Such vectors may comprise further genes such as marker genes which allow for the selection of said vector in a suitable host cell and under suitable conditions.
Preferably, the polynucleotide of the invention is operatively linked to expression control sequences allowing expression in prokaryotic or eukaryotic cells. Expression of said polynucleotide comprises transcription of the polynucleotide into a translatable mRNA. Regulatory elements ensuring expression in eukaryotic cells, preferably mammalian cells, are well known to those skilled in the art. They usually comprise regulatory sequences ensuring initiation of transcription and optionally poly-A signals ensuring termination of transcription and stabilization of the transcript. Additional regulatory elements may include transcriptional as well as translational enhancers, and/or naturally associated or heterologous promoter regions.
In this respect, the person skilled in the art will readily appreciate that the polynucleotides encoding at least the variable domain of the light and/or heavy chain may encode the variable domains of both immunoglobulin chains or only one. Likewise, said polynucleotides may be under the control of the same promoter or may be separately controlled for expression. Possible regulatory elements permitting expression in prokaryotic host cells comprise, e.g., the PL, lac, trp or tac promoter in E. coli, and examples for regulatory elements permitting expression in eukaryotic host cells are the AOX1 or GAL1 promoter in yeast or the CMV-, SV40- , RSV-promoter (Rous sarcoma virus), CMV-enhancer, SV40-enhancer or a globin intron in mammalian and other animal cells.
Beside elements which are responsible for the initiation of transcription such regulatory elements may also comprise transcription termination signals, such as the SV40-poly-A site or the tk-poly-A site, downstream of the polynucleotide. Furthermore, depending on the expression system used leader sequences capable of directing the polypeptide to a cellular compartment or secreting it into the medium may be added to the coding sequence of the polynucleotide of the invention and are well known in the art. The leader sequence(s) is (are) assembled in appropriate phase with translation, initiation and termination sequences, and preferably, a leader sequence capable of directing secretion of translated protein, or a portion thereof, into the periplasmic space or extracellular medium. Optionally, the heterologous sequence can encode a fusion protein including a C- or N-terminal identification peptide imparting desired characteristics, e.g., stabilization or simplified purification of expressed recombinant product. In this context, suitable expression vectors are known in the art such as Okayama-Berg cDNA expression vector pcDV1 (Pharmacia), pCDM8, pRc/CMV, pcDNA1, pcDNA3 (Invitrogen), or pSPORT1 (GIBCO BRL).
Preferably, the expression control sequences will be eukaryotic promoter systems in vectors capable of transforming or transfecting eukaryotic host cells, but control sequences for prokaryotic hosts may also be used. Once the vector has been incorporated into the appropriate host, the host is maintained under conditions suitable for high level expression of the nucleotide sequences, and, as desired, the collection and purification of the immunoglobulin light chains, heavy chains, light/heavy chain dimers or intact antibodies, binding fragments or other immunoglobulin forms may follow; see, Beychok, Cells of Immunoglobulin Synthesis, Academic Press, N.Y., (1979).

As described above, the polynucleotide of the invention can be used alone or as part of a vector to express the (poly)peptide of the invention in cells, for, e.g., gene therapy or diagnostics of diseases related to immune diseases. The polynucleotides or vectors of the invention are introduced into the cells which in turn produce the antibody. Gene therapy, which is based on introducing therapeutic genes into cells by ex-vivo or in-vivo techniques is one of the most important applications of gene transfer. Suitable vectors and methods for in-vitro or in-vivo gene therapy are described in the literature and are known to the person skilled in the art; see, e.g., Giordano, Nature Medicine 2 (1996), 534-539; Schaper, Circ. Res. 79 (1996), 911-919; Anderson, Science 256 (1992), 808-813; Isner, Lancet 348 (1996), 370-374; Muhlhauser, Circ. Res. 77 (1995), 1077-1086; Wang, Nature Medicine 2 (1996), 714-716; WO94/29469; WO 97/00957 or Schaper, Current Opinion in Biotechnology 7 (1996), 635-640, and references cited therein. The polynucleotides and vectors of the invention may be designed for direct introduction or for introduction via liposomes, or viral vectors (e.g. adenoviral, retroviral) into the cell. Preferably, said cell is a germ line cell, embryonic cell, or egg cell or derived therefrom, most preferably said cell is a stem cell.

Furthermore, the present invention relates to vectors, particularly plasmids, cosmids, viruses and bacteriophages used conventionally in genetic engineering that comprise a polynucleotide encoding a variable domain of an immunoglobulin chain of an antibody of the invention; optionally in combination with a polynucleotide of the invention that encodes the variable domain of the other immunoglobulin chain of the antibody of the invention. Preferably, said vector is an expression vector and/or a gene transfer or targeting vector. Expression vectors derived from viruses such as retroviruses, vaccinia virus, adeno-associated virus, herpes viruses, or bovine papilloma virus, may be used for delivery of the polynucleotides or vector of the invention into targeted cell population. Methods which are well known to those skilled in the art can be used to construct recombinant viral vectors; see, for example, the techniques described in Sambrook, Molecular Cloning A Laboratory Manual, Cold Spring Harbor Laboratory (1989) N.Y. and Ausubel, Current Protocols in Molecular Biology, Green Publishing Associates and Wiley Interscience, N.Y. (1994). Alternatively, the polynucleotides and vectors of the invention can be reconstituted into liposomes for delivery to target cells. The vectors containing the polynucleotides of the invention (e.g., the heavy and/or light variable domain(s) of the immunoglobulin chains encoding sequences and expression control sequences) can be transferred into the host cell by well known methods, which vary depending on the type of cellular host. For example, calcium chloride transfection is commonly utilized for prokaryotic cells, whereas calcium phosphate treatment or electroporation may be used for other cellular hosts; see Sambrook, supra.

The present invention furthermore relates to host cells transformed with a polynucleotide or vector of the invention. Said host cell may be a prokaryotic or eukaryotic cell. The polynucleotide or vector of the invention which is present in the host cell may either be integrated into the genome of the host cell or it may be maintained extrachromosomally. The host cell can be any prokaryotic or eukaryotic cell, such as a bacterial, insect, fungal, plant, animal or human cell. Preferred fungal cells are, for example, those of the genus Saccharomyces, in particular those of the species S. cerevisiae. The term "prokaryotic" is meant to include all bacteria which can be transformed or transfected with a DNA or RNA molecules for the expression of an antibody of the invention or the corresponding immunoglobulin chains. Prokaryotic hosts may include gram negative as well as gram positive bacteria such as, for example, E. coli, S. typhimurium, Serratia marcescens and Bacillus subtilis. The term "eukaryotic" is meant to include yeast, higher plant, insect and preferably mammalian cells, most preferably NSO and CHO cells. Depending upon the host employed in a recombinant production procedure, the antibodies or immunoglobulin chains encoded by the polynucleotide of the present invention may be glycosylated or may be non-glycosylated. Antibodies of the invention or the corresponding immunoglobulin chains may also include an initial methionine amino acid residue. A polynucleotide of the invention can be used to transform or transfect the host using any of the techniques commonly known to those of ordinary skill in the art. Furthermore, methods for preparing fused, operably linked genes and expressing them in, e.g., mammalian cells and bacteria are well-known in the art (Sambrook, Molecular Cloning: A Laboratory Manual, Cold Spring Harbor Laboratory, Cold Spring Harbor, NY, 1989). The genetic constructs and methods described therein can be utilized for expression of the antibody of the invention or the corresponding immunoglobulin chains in eukaryotic or prokaryotic hosts. In general, expression vectors containing promoter sequences which facilitate the efficient transcription of the inserted polynucleotide are used in connection with the host. The expression vector typically contains an origin of replication, a promoter, and a terminator, as well as specific genes which are capable of providing phenotypic selection of the transformed cells. Suitable source cells for the DNA sequences and host cells for immunoglobulin expression and secretion can be obtained from a number of sources, such as the American Type Culture Collection ("Catalogue of Cell Lines and Hybridomas," Fifth edition (1985) Rockville, Maryland, U.S.A., which is incorporated herein by reference). Furthermore, transgenic animals, preferably mammals, comprising cells of the invention may be used for the large scale production of the antibody of the invention.

Thus, in a further embodiment, the present invention relates to a method for the production of an antibody capable of modulating T cell proliferation in an allogenic mixed lymphocyte reaction and/or suppressing the cytolytic activity of intestinal intraepithelial lymphocytes (iIELs) or a functional fragment or immunoglobulin chain(s) thereof, said method comprising
(a) culturing a cell described above; and
(b) isolating said antibody or functional fragment or immunoglobulin chain(s) thereof from the culture.

The transformed hosts can be grown in fermentors and cultured according to techniques known in the art to achieve optimal cell growth. Once expressed, the whole antibodies, their dimers, individual light and heavy chains, or other immunoglobulin forms of the present invention, can be purified according to standard procedures of the art, including ammonium sulfate precipitation, affinity columns, column chromatography, gel electrophoresis and the like; see, Scopes, "Protein Purification", Springer Verlag, N.Y. (1982). The antibody or its corresponding immunoglobulin chain(s) of the invention can then be isolated from the growth medium, cellular lysates, or cellular membrane fractions. The isolation and purification of the, e.g., microbially expressed antibodies or immunoglobulin chains of the invention may be by any conventional means such as, for example, preparative chromatographic separations and immunological separations such as those involving the use of monoclonal or polyclonal antibodies directed, e.g., against the constant region of the antibody of the invention. It will be apparent to those skilled in the art that the antibodies of the invention can be further coupled to other moieties for, e.g., drug targeting and imaging applications. Such coupling may be conducted chemically after expression of the antibody or antigen to site of attachment or the coupling product may be engineered into the antibody or antigen of the invention at the DNA level. The DNAs are then expressed in a suitable host system, and the expressed proteins are collected and renatured, if necessary.
Substantially pure immunoglobulins of at least about 90 to 95% homogeneity are preferred, and 98 to 99% or more homogeneity most preferred, for pharmaceutical uses. Once purified, partially or to homogeneity as desired, the antibodies may then be used therapeutically (including extracorporeally) or in developing and performing assay procedures.

The present invention also involves a method for producing cells capable of expressing an antibody of the invention or its corresponding immunoglobulin chain(s) comprising genetically engineering cells with the polynucleotide or with the vector of the invention. The cells obtainable by the method of the invention can be used, for example, to test the interaction of the antibody of the invention with its antigen.

As mentioned before, the immunoglobulin or its encoding cDNAs may be further modified. Thus, in a further embodiment the method of the present invention comprises any one of the step(s) of producing a chimeric antibody, humanized antibody, single-chain antibody, Fab-fragment, bi-specific antibody, fusion antibody, labelled antibody or an analog of any one of those. Corresponding methods are known to the person skilled in the art and are described, e.g., in Harlow and Lane "Antibodies, A Laboratory Manual", CSH Press, Cold Spring Harbor, 1988. When derivatives of said antibodies are obtained by the phage display technique, surface plasmon resonance as employed in the BIAcore system can be used to increase the efficiency of phage antibodies which bind to the same epitope as that of any one of the antibodies described herein (Schier, Human Antibodies Hybridomas 7 (1996), 97-105; Malmborg, J. Immunol. Methods 183 (1995), 7-13). The production of chimeric antibodies is described, for example, in WO89/09622. Methods for the production of humanized antibodies are described in, e.g., EP-A1 0 239 400 and WO90/07861. A further source of antibodies to be utilized in accordance with the present invention are so-called xenogeneic antibodies. The general principle for the production of xenogeneic antibodies such as human antibodies in mice is described in, e.g., WO 91/10741, WO 94/02602, WO 96/34096 and WO 96/33735. As discussed above, the antibody of the invention may exist in a variety of forms besides complete antibodies; including, for example, Fv, Fab and F(ab)₂, as well as in single chains; see e.g. WO88/09344.

The antibodies of the present invention or their corresponding immunoglobulin chain(s) can be further modified using conventional techniques known in the art, for example, by using amino acid deletion(s), insertion(s), substitution(s), addition(s), and/or recombination(s) and/or any other modification(s) known in the art either alone or in combination. Methods for introducing such modifications in the DNA sequence underlying the amino acid sequence of an immunoglobulin chain are well known to the person skilled in the art; see, e.g., Sambrook, Molecular Cloning A Laboratory Manual, Cold Spring Harbor Laboratory (1989) N.Y. and Ausubel, Current Protocols in Molecular Biology, Green Publishing Associates and Wiley Interscience, N.Y. (1994). Modifications of the antibody of the invention include chemical and/or enzymatic derivatizations at one or more constituent amino acids, including side chain modifications, backbone modifications, and N- and C-terminal modifications including acetylation, hydroxylation, methylation, amidation, and the attachment of carbohydrate or lipid moieties, cofactors, and the like. Likewise, the present invention encompasses the production of chimeric proteins which comprise the described antibody or some fragment thereof at the amino terminus fused to heterologous molecule such as an immunostimulatory ligand at the carboxyl terminus; see, e.g., WO00/30680 for corresponding technical details.

Additionally, the present invention encompasses small polypeptides including those containing a BGP binding fragment as described above, for example containing the CDR3 region of the variable region of any one of the mentioned monoclonal antibodies. Such peptides may easily be synthesized or produced by recombinant means to produce a BGP binding agent useful according to the invention. Such methods are well known to those of ordinary skill in the art. Peptides can be synthesized for example, using automated peptide synthesizers which are commercially available. The peptides can be produced by recombinant techniques by incorporating the DNA expressing the peptide into an expression vector and transforming cells with the expression vector to produce the peptide. The sequence of the CDR regions, for use in synthesizing peptide BGP binding agents, may be determined by methods known in the art. The heavy chain variable region is a peptide which generally ranges from 100 to 150 amino acids in length. The light chain variable region is a peptide which generally ranges from 80 to 130 amino acids in length. The CDR sequences within the heavy and light chain variable regions which include only approximately 3-25 amino acids may easily be sequenced by one of ordinary skill in the art. The peptides may even be synthesized by commercial sources.
To determine whether a peptide binds to BGP any known binding assay may be employed. For example, the peptide may be immobilized on a surface and then contacted with labeled BGP. The amount of BGP which interacts with the peptide or the amount which does not bind to the peptide may then be quantitated to determine whether the peptide binds to BGP. A surface having the aforementioned anti-BGP monoclonal antibodies immobilized thereto may serve as a positive control.

Screening of BGP binding agents also can be carried out utilizing a competition assay. If the BGP binding agent being tested competes with an anti-BGP monoclonal antibody, as shown by a decrease in binding of the monoclonal antibody, then it is likely that the agent and the anti-BGP monoclonal antibody bind to the same, or a closely related, epitope. Still another way to determine whether an agent has the specificity of the anti-BGP monoclonal antibodies described above is to pre-incubate the monoclonal antibody with BGP with which it is normally reactive (i.e., binds), and then add the agent being tested to determine if the agent being tested is inhibited in its ability to bind BGP. If the agent being tested is inhibited then, in all likelihood, it has the same or a functionally equivalent epitope and specificity as the anti-BGP monoclonal antibodies.

Using routine procedures known to those of ordinary skill in the art, one can determine whether a BGP binding agent is useful according to the invention by determining whether the agent is one which modulates T cell proliferation or cytotoxicity in an in vitro assay such as measuring release of TNF from T cells or by ⁵¹Cr release assay; see, e.g., Herin et al., Int. J. Cancer 39 (1987), 390-396. Other assays are described in the Examples and elsewhere herein.

The polypeptides (e. g. antibodies) and other BGP binding agents described above can also be used immunotherapeutically for T cell sensitive disorders in humans. The term "immunotherapeutically" or "immunotherapy" as used herein in conjunction with the BGP binding agents denotes both prophylactic as well as therapeutic administration. Thus, the peptides can be administered to high-risk subjects in order to lessen the likelihood and/or severity of a T cell sensitive disease, such as a tumor, transplant rejection or autoimmune disease, or administered to subjects already evidencing such diseases.

BGP binding agents which increase or decrease T cell activity can be selected using the assays described, for example, in WO99/52552 and according to standard T cell cytotoxicity and proliferation assays, such as mixed lymphocyte reactions, chromium release assays, TNF release assays, and thymidine incorporation assays. It is believed that a monovalent BGP binding agent, for example derivatives of single chain antibodies which have only one binding domain of the original antibody, will inhibit the inhibitory signal of BGP by reducing cross-linking of BGP polypeptides expressed by T cells, and that a multivalent BGP binding agent (having two or more BGP binding sites), such as bi-an tri-specific antibodies, will increase the inhibitory signal of BGP in T cells by increasing cross-linking of BGP polypeptides expressed by T cells.

Hence, the present invention relates to any antibody and similar binding molecules, which preferably have substantially the same immunological binding characteristics as monoclonal antibodies 34B1, 26H7 or 5F4, i.e. which recognize the same epitope and with substantially the same affinity, or at least 1/10 of the affinity as the antibodies of the invention exemplified herein. Such antibodies and binding molecules can be tested for their binding specificity and affinity by for example by using competitive assays with an antibody produced by a hybridoma of the invention. The antibodies of the present invention will typically find use individually in treating substantially any disease susceptible to monoclonal antibody based therapy. In particular, the immunoglobulins can be used as immunosuppressive agents. For an antibody of the invention, typical disease states suitable for treatment include inflammatory symptoms. The antibodies can be used therapeutically in, e.g., patients suffering an diseases related to immune response; see supra. Such therapy can be accomplished by, for example, the administration of antibodies of the invention.
Such administration can utilize unlabeled as well as labeled antibodies or antigens. Labeling agents can be coupled either directly or indirectly to the antibodies or antigens of the invention. One example of indirect coupling is by use of a spacer moiety. Furthermore, the antibodies of the present invention can comprise a further domain, said domain being linked by covalent or non-covalent bonds. The linkage can be based on genetic fusion according to the methods known in the art and described above or can be performed by, e.g., chemical cross-linking as described in, e.g., WO 94/04686. The additional domain present in the fusion protein comprising the antibody of the invention may preferably be linked by a flexible linker, advantageously a polypeptide linker, wherein said polypeptide linker comprises plural, hydrophilic, peptide-bonded amino acids of a length sufficient to span the distance between the C-terminal end of said further domain and the N-terminal end of the antibody of the invention or vice versa. The above described fusion protein may further comprise a cleavable linker or cleavage site for proteinases. These spacer moieties, in turn, can be either insoluble or soluble (Diener et al., Science 231 (1986), 148) and can be selected to enable drug release from the antigen at the target site. Examples of therapeutic agents which can be coupled to the antibodies, antigens and epitopes of the invention for immunotherapy are drugs, radioisotopes, lectins, and toxins. The drugs with which can be conjugated to the antibodies, antigens and epitopes of the invention include compounds which are classically referred to as drugs such as mitomycin C, daunorubicin, and vinblastine. In using radioisotopically conjugated antibodies, antigens or epitopes of the invention for, e.g., immunotherapy, certain isotopes may be more preferable than others depending on such factors as leukocyte distribution as well as stability and emission. Depending on the autoimmune response, some emitters may be preferable to others. In general, a and b particle emitting radioisotopes are preferred in immunotherapy. Preferred are short range, high energy a emitters such as ²¹²Bi. Examples of radioisotopes which can be bound to the antibodies, antigens or epitopes of the invention for therapeutic purposes are ¹²⁵I, ¹³¹I, ⁹⁰Y, ⁶⁷Cu, ²¹²Bi, ²¹²At, ²¹¹Pb, ⁴⁷Sc, ¹⁰⁹_{Pd} and ¹⁸⁸Re. Other therapeutic agents which can be coupled to the antibody, antigen or epitope of the invention, as well as ex vivo and in vivo therapeutic protocols, are known, or can be easily ascertained, by those of ordinary skill in the art. Wherever appropriate the person skilled in the art may use a polynucleotide of the invention encoding any one of the above described antibodies, antigens or the corresponding vectors instead of the proteinaeous material itself.

Moreover, the present invention relates to compositions comprising the aforementioned antibody or antigen-binding fragment of the invention or chemical derivatives thereof, or the polynucleotide, vector or cell of the invention. The composition of the present invention may further comprise a pharmaceutically acceptable carrier. The term "chemical derivative" describes a molecule that contains additional chemical moieties that are not normally a part of the base molecule. Such moieties may improve the solubility, half-life, absorption, etc. of the base molecule. Alternatively the moieties may attenuate undesirable side effects of the base molecule or decrease the toxicity of the base molecule. Examples of such moieties are described in a variety of texts, such as Remington's Pharmaceutical Sciences. Examples of suitable pharmaceutical carriers are well known in the art and include phosphate buffered saline solutions, water, emulsions, such as oil/water emulsions, various types of wetting agents, sterile solutions etc. Compositions comprising such carriers can be formulated by well known conventional methods. These pharmaceutical compositions can be administered to the subject at a suitable dose. Administration of the suitable compositions may be effected by different ways, e.g., by intravenous, intraperitoneal, subcutaneous, intramuscular, topical or intradermal administration. Aerosol formulations such as nasal spray formulations include purified aqueous or other solutions of the active agent with preservative agents and isotonic agents. Such formulations are preferably adjusted to a pH and isotonic state compatible with the nasal mucous membranes. Formulations for rectal or vaginal administration may be presented as a suppository with a suitable carrier.
The dosage regimen will be determined by the attending physician and clinical factors. As is well known in the medical arts, dosages for any one patient depends upon many factors, including the patient's size, body surface area, age, the particular compound to be administered, sex, time and route of administration, general health, and other drugs being administered concurrently. A typical dose can be, for example, in the range of 0.001 µg to 10 mg (or of nucleic acid for expression or for inhibition of expression in this range); however, doses below or above this exemplary range are envisioned, especially considering the aforementioned factors. Generally, the regimen as a regular administration of the pharmaceutical composition should be in the range of 0.01 µg to 10 mg units per day. If the regimen is a continuous infusion, it should also be in the range of 0.01 µg to 10 mg units per kilogram of body weight per minute, respectively. Progress can be monitored by periodic assessment. Dosages will vary but a preferred dosage for intravenous administration of DNA is from approximately 10⁶ to 10¹² copies of the DNA molecule.

The compositions of the invention may be administered locally or systemically. Administration will generally be parenterally, e.g., intravenously; DNA may also be administered directly to the target site, e.g., by biolistic delivery to an internal or external target site or by catheter to a site in an artery. Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, and inert gases and the like.

Furthermore, the pharmaceutical composition of the invention may comprise further agents such as interleukins or interferons depending on the intended use of the pharmaceutical composition.

In a preferred embodiment, the pharmaceutical composition of the present invention comprises at least one second agent, preferably an agent which inhibits T-cell stimulation depending on the intended use. Such agents include, for example, molecules that are capable of blocking or mimicking receptor/ligand interaction or the like which leads to T-cell suppression. Such agents comprise those blocking the activity of, e.g., costimulatory molecules, such as anti-TIRC7 antibodies, anti-TNF-α antibodies, integrins, Ig-superfamily molecules, selectins as well as drugs blocking chemokines and their respective receptor interactions, drugs blocking IL2/IL2-receptor interaction and other conventional immunosuppressive drugs such as IL-2R monoclonal antibodies, IL-Toxins and IL-Muteins. Examples for costimulatory molecules and their ligands are described in the prior art, e.g., in Schwartz, Cell 71 (1992), 1065-1068. The interruption of the receptor/ligand interactions by using monoclonal antibodies or soluble CTLA4Ig for the interaction between CD28 to the B7-2 and CTLA4 to B7-1 and B7-2 are described in Blazar, J. Immunol. 157 (1996), 3250-3259; Bluestone, Immunity 2 (1995), 555-559; Linsley, Science 257 (1992), 792-95. Examples for blocking the receptor/ligand interaction by using monoclonal antibodies to CD40 or CD40L are reported by Burden, Nature 381 (1996), 434-435; Kirk, Proc. Natl. Acad. Sci. USA 94 (1997), 8789-8794. CD2 antigen and its ligand LFA-3 are described in Bagogui Li et al., review in Adhesion Molecules, Fusion proteins, Novel Peptides, and Monoclonal Antibodies, Recent Developments in Transplantation Medicine, Vol. II, 1995, Physicians&Scientists Publishing Co., Inc. and blocking of their interaction by using of monoclonal antibodies (anti-Leu-5b, OKT11, T11) is reported in Bromberg, Transplantation 51 (1991) 219-225 or CD2.1gG1 fusion protein. The use of monoclonal Abs agains CD4 molecule is described in Cosimi, Surgery 108 (1990), 406-414. CD47 blockade by monoclonal antibodies is described by Rheinhold, J. Exp. Med. 185 (1997), 1-11. Integrins and Ig-superfamily molecules include LFA-1 with its ligand ICAM-1, -2, -3, Mac-1 with its ligand ICAM-1, -3; ICAM-1 with its ligand LFA-1, Mac-1, CD43; ICAM-2 with its ligand LFA-1; ICAM-3 with its ligand LFA-1, Mac-1; VLA4 and VCAM-1 see, e.g., David, Adams, review in Adhesion Molecules, Fusion proteins, Novel Peptides, and Monoclonal Antibodies, Recent Developments in Transplantation Medicine, Vol. II, 1995, Physicians&Scientists Publishing Co., Inc.; Isobe, Science, 255 (1992), 1125-1127; Cosimi, J. Immunology 144 (1990), 4604-4612; Hynes, Cell 69 (1992),11-25.
Furthermore selectively interfering agents with VLA-4 monoclonal antibodies to the alpha4 integrin chain (CD49d) can be used, beta1 integrin chain (CD29), or an activation-induced neo-epitope of VLA-4 as well as soluble VLA-4 ligands such as soluble fibronectin or its relevant peptide (GPEILDVPST), or soluble VCAM-1 or its relevant peptide. More selectively blocking agents are antisense oligonucleotides, designed to selectively hybridize with cytoplasmic alpha4, beta1, or VCAM-1 mRNA; Fedoseyeva, J. Immunol. 57 (1994), 606-612. Another example is the drug pentoxifylline (PTX) that is able to block expression of VCAM-1; Besler, J. Leukoc. Biol. 40 (1986), 747-754. Furthermore, VCAM-1 monoclonal antibody, M/K-2, anti-murine, for example could prolong allograft survival, Orosz, Transplantation, 56 (1993), 453-460. Blocking of members of the integrin family and their ligands by using monoclonal antibodies is decribed in Kupiec-Weglinski, review in Adhesion Molecules, Fusion proteins, Novel Peptides, and Monoclonal Antibodies, Recent Developments in Transplantation Medicine, Vol. II, 1995, Physicians&Scientists Publishing Co., Inc. Selectins, e.g., L-selectin (CD62L), E-selectin (CD62E), P-selectin (CD62P) have been described in Forrest and Paulson, Selectin family of adhesion molecules. In: Granger and Schmid-Schonbein, eds. Physiology and Pathophysiology of Leukocyte Adhesion. New York, Oxford Press, 1995, pp 68-146. The combination of conventional immunosuppressive drugs, e.g., ATG, ALG, OKT3, Azathioprine, Mycophenylate, Mofetyl, Cyclosporin A, FK506, Sirolimus (Rapamune), Corticosteroids may be used as described in Cosimi, Transplantation 32 (1981), 535-539; Shield, Transplantation 38 (1984), 695-701, and Graft, June 2001, Vol 4 (4). The interruption of chemokines and interactions with their respective receptor by using monoclonal antibodies is reviewed in Luster, Chemokines-chemotactic cytokines that mediate inflammation, New Engl. J. Med. Feb. (1998), 436-445. Thus, any agent as defined above and referenced by way of example can be used in accordance with the pharmaceutical composition of the invention or the methods and uses described herein.

Therapeutic or diagnostic compositions of the invention are administered to an individual in a effective dose sufficient to treat or diagnose disorders in which modulation of BGP-related activity is indicated. The effective amount may vary according to a variety of factors such as the individual's condition, weight, sex and age. Other factors include the mode of administration. The pharmaceutical compositions may be provided to the individual by a variety of routes such as by intracoronary, intraperitoneal, subcutaneous, intravenous, transdermal, intrasynovial, intramuscular or oral routes. In addition, coadministration or sequential administration of other agents may be desirable.
A therapeutically effective dose refers to that amount of antibodies, antigen-binding fragments, polynucleotides and vectors of the invention ameliorate the symptoms or condition. Therapeutic efficacy and toxicity of such compounds can be determined by standard pharmaceutical procedures in cell cultures or experimental animals, e.g., ED50 (the dose therapeutically effective in 50% of the population) and LD50 (the dose lethal to 50% of the population). The dose ratio between therapeutic and toxic effects is the therapeutic index, and it can be expressed as the ratio, LD50/ED50.

Thus, the present invention relates to the use of the antibody and the antigen of the invention for the preparation of a pharmaceutical composition for inhibition of an immune response, preferably for the treatment of graft versus host disease, autoimmune diseases, allergic diseases, infectious diseases, sepsis, for the treatment of tumors, for the improvement of wound healing or for inducing or maintaining immune unresponsiveness in a subject wherein in certain circumstances one would want to inhibit (e.g. GVHD, etc.) or enhance (e.g. anti-tumor response) T cell activity.

Accordingly, the present invention also relates to a method of modulating the immune response in a subject in need thereof, comprising administering the antibody or antigen-binding fragment of the invention. Compositions comprising the antibody or the antigen-binding fragment of this invention can be added to cells in culture (in vitro) or used to treat patients, such as mammals (in vivo). Where the antibody or the antigen are used to treat a patient, the polypeptide is preferably combined in a pharmaceutical composition with a pharmaceutically acceptable carrier such as a larger molecule to promote polypeptide stability or a pharmaceutically acceptable buffer that serves as a carrier for the antibodies that has more than one antibody coupled to a single entity. The methods of the invention include administering to a patient, preferably a mammal, and more preferably a human, the composition of the invention in an amount effective to produce the desired effect. The antibody or the antigen can be administered as a single dose or in multiple doses. Useful dosages of the active agents can be determined by comparing their in vitro activity and the in vivo activity in animal models. Methods for extrapolation of effective dosages in mice, and other animals, to humans are known in the art. The present invention also provides a method of modulating (e.g., activating or inhibiting) immune cell, preferably T lymphocytes, activation, proliferation, and/or differentiation that includes contacting an immune cell with an antibody or the antigen-binding fragment described above. Given the fact that C-CAM1 is expressed by dendritic cells at least in mouse (and probably in human), the antibodies of the present invention may disrupt the initiation of an immune response in addition to arresting an ongoing immune response.

From the foregoing, it is evident that the present invention encompasses any use of a ligand binding molecule comprising at least one CDR of the above described antibody, in particular for diagnosing and/or treatment of a disorder related to the expression or malfunction of BGP(C-CAM1). Preferably, said ligand binding molecule is an antibody of the present invention or an immunoglobulin chain thereof. In addition, the present invention relates to anti-idiotypic antibodies of the any one of the mentioned monoclonal antibodies described hereinbefore. These are antibodies or other binding molecules which bind to the unique antigenic peptide sequence located on an antibody's variable region near the antigen binding site.

The biological activity of the antibodies identified here suggests that they have sufficient affinity to make them potential candidates for drug localization to cells expressing the appropriate surface structures. This targeting and binding to cells could be useful for the delivery of therapeutically or diagnostically active agents (including targeting drugs, DNA sequences, RNA sequences, lipids, proteins (e.g., human growth factors)) and gene therapy/gene delivery. More preferably, the therapeutically active agent is an antiinflammatory agent. Molecules/particles with an antibody of the invention would bind specifically to cells/tissues expressing BGP, and therefore could have diagnostic and therapeutic use. Thus, the antibody or the antigen of the present invention can be labeled (e.g., fluorescent, radioactive, enzyme, nuclear magnetic) and used to detect specific targets in vivo or in vitro including "immunochemistry" like assays in vitro. In vivo they could be used in a manner similar to nuclear medicine imaging techniques to detect tissues, cells, or other material expressing BGP, in particular the N-domain thereof. Another method involves delivering a therapeutically active agent to a patient. The method includes administering at least one antibody or the antigen-binding fragment and the therapeutically active agent to a patient. Preferably, the therapeutically active agent is selected from drugs, DNA sequences, RNA sequences, proteins, lipids, and combinations thereof. More preferably, the therapeutically active agent is an antibacterial agent, antiinflammatory agent, or antineoplastic agent.
The therapeutically or diagnostically active agent can be coupled to the antibody of the invention or an antigen-binding fragment thereof by various means. This includes, for example, single-chain fusion proteins comprising the variable regions of the antibody of the invention coupled by covalent methods, such as peptide linkages, to the therapeutically or diagnostically active agent. Further examples include molecules which comprise at least an antigen-binding fragment coupled to additional molecules covalently or non-covalently include those in the following non-limiting illustrative list. Traunecker, Int. J. Cancer Surp. SuDP 7 (1992), 51-52, describe the bispecific reagent janusin in which the Fv region directed to CD3 is coupled to soluble CD4 or to other ligands such as OVCA and IL-7. Similarly, the variable regions of the antibody of the invention can be constructed into Fv molecules and coupled to alternative ligands such as those illustrated in the cited article. Higgins, J. Infect Disease 166 (1992), 198-202, described a hetero-conjugate antibody composed of OKT3 cross-linked to an antibody directed to a specific sequence in the V3 region of GP120. Such hetero-conjugate antibodies can also be constructed using at least the variable regions contained in the antibody of the invention methods. Additional examples of specific antibodies include those described by Fanger, Cancer Treat. Res. 68 (1993), 181-194 and by Fanger, Crit. Rev. Immunol. 12 (1992), 101-124. Conjugates that are immunotoxins including conventional antibodies have been widely described in the art. The toxins may be coupled to the antibodies by conventional coupling techniques or immunotoxins containing protein toxin portions can be produced as fusion proteins. The antibodies of the present invention can be used in a corresponding way to obtain such immunotoxins. Illustrative of such immunotoxins are those described by Byers, Seminars Cell. Biol. 2 (1991), 59-70 and by Fanger, Immunol. Today 12 (1991), 51-54.

In another embodiment the present invention relates to a diagnostic composition comprising any one of the above described antibodies, antigen-binding fragments, polynucleotides, vectors or cells of the invention and optionally suitable means for detection. The antibodies of the invention are, for example, suited for use in immunoassays in which they can be utilized in liquid phase or bound to a solid phase carrier. Examples of immunoassays which can utilize the antibody of the invention are competitive and non-competitive immunoassays in either a direct or indirect format. Examples of such immunoassays are the radioimmunoassay (RIA), the sandwich (immunometric assay), flow cytometry and the Western blot assay. The antigens and antibodies of the invention can be bound to many different carriers and used to isolate cells specifically bound to said polypeptides. Examples of well known carriers include glass, polystyrene, polyvinyl chloride, polypropylene, polyethylene, polycarbonate, dextran, nylon, amyloses, natural and modified celluloses, polyacrylamides, agaroses, and magnetite. The nature of the carrier can be either soluble or insoluble for the purposes of the invention. There are many different labels and methods of labeling known to those of ordinary skill in the art. Examples of the types of labels which can be used in the present invention include enzymes, radioisotopes, colloidal metals, fluorescent compounds, chemiluminescent compounds, and bioluminescent compounds; see also the embodiments discussed hereinabove.

By a further embodiment, the antibodies of the invention may also be used in a method for the diagnosis of BGP-related diseases in an individual by obtaining a body fluid sample from the tested individual which may be a blood sample, a lymph sample or any other body fluid sample and contacting the body fluid sample with an antibody of the invention under conditions enabling the formation of antibody-antigen complexes. The level of such complexes is then determined by methods known in the art, a level significantly higher than that formed in a control sample indicating the disease in the tested individual. In the same manner, the specific antigen bound by the antibodies of the invention may also be used. Thus, the present invention relates to an in vitro immunoassay comprising the antibody or the antigen of the invention.

These and other embodiments are disclosed and encompassed by the description and Examples of the present invention. Further literature concerning any one of the antibodies, methods, uses and compounds to be employed in accordance with the present invention may be retrieved from public libraries and databases, using for example electronic devices. For example the public database "Medline" may be utilized, which is hosted by the National Center for Biotechnology Information and/or the National Library of Medicine at the National Institutes of Health. Further databases and web addresses, such as those of the European Bioinformatics Institute (EBI), which is part of the European Molecular Biology Laboratory (EMBL) are known to the person skilled in the art and can also be obtained using internet search engines. An overview of patent information in biotechnology and a survey of relevant sources of patent information useful for retrospective searching and for current awareness is given in Berks, TIBTECH 12 (1994), 352-364.

The above disclosure generally describes the present invention. A more complete understanding can be obtained by reference to the following specific examples which are provided herein for purposes of illustration only and are not intended to limit the scope of the invention.

### Examples

The examples which follow further illustrate the invention, but should not be construed to limit the scope of the invention in any way. Detailed descriptions of conventional methods, such as those employed in the construction of vectors and plasmids, the insertion of genes encoding polypeptides into such vectors and plasmids, the introduction of plasmids into host cells, and the expression and determination thereof of genes and gene products can be obtained from numerous publication, including Sambrook et al., (1989) Molecular Cloning: A Laboratory Manual, 2nd ed., Cold Spring Harbor Laboratory Press. Particularly useful means and methods such as cells and cell lines, BGP molecules, flow cytometry, radiolabeling, immunoprecipitation and electrophoresis, production of soluble recombinant proteins, etc. are described in WO99/52552. All references mentioned herein are incorporated in their entirety.

### EXAMPLE 1: Hybridomas that secrete BGP(C-CAM1) specific antibodies

The 34B1, 26H7 and 5F4 monoclonal antibodies (mAbs) were produced by immunizing BALB/c mice with the activated human mucosal lymphocyte line, 191 E, as previously described (Russell et al., J. Immunol. 157 (1996), 3366-3374). Three intraperitoneal injections and a final intravenous injection of 5 x 10⁶ lymphocytes were given at two-week intervals. Three days after the intravenous immunization, splenocytes were isolated and fused with NS 1 murine myeloma cells in the presence of PEG (m. w. 1450) as described previously (Cerf-Bensussan et al., Eur. J. Immunol. 17 (1987), 1279). Hybridomas were selected with aminopterin-containing medium, and hybridoma supernatants were screened by indirect immunoperoxidase staining of frozen intestinal and tonsillar tissue sections. Positive hybridomas were subcloned twice by limiting dilution, and ascites containing the antibody was produced by intraperitoneal injection of the hybridoma cells into pristane-treated BALB/c mice.

The isotypes of 34B1 (IgG1), 26H7 (IgG1) and 5F4 (IgG1) were determined by ELISA using murine isotype-specific mAb (Boehringer Mannheim, Indianapolis, IN). W6/32 is a mouse IgG2a mAb specific for human MHC class I (kindly provided by Dr. Jack Strom-inger, Dana Farber Cancer Institute, Boston, MA). OKT3 (IgG2a is a mouse anti-human CD3 mAb (kindly provided by Dr. Robert Finberg, Dana-Farber Cancer Institute, Boston, MA). TS2/18 (kindly provided by Dr. Lloyd Klickstein, Brigham and Women's Hospital, Boston, MA) is an anti-CD2 mAb (mouse IgG2a). OKT11 (kindly provided by Dr. Ellis Reionherz, Dana-Farber Cancer Institute) is an anti-CD2 mAb (mouse IgG29). OKT4 and OKT8 are mouse IgG2a mAbs specific for human CD4 and CD8-oc, respectively (obtained from American Type Culture Collection, Bethesda, MD). UCHT1, directly conjugated to phycoerythrin, is a mouse IgGI mAb specific for human CD3-E (Dako, Denmark). The MA22 (CD66a; clone YG-C94G7; IgG1), MA26 (CD66a; clone 4.3.17; IgG1), MA27 (CD66e; clone 26/5/1; IgG2a), MA28 (CD66e; clone 26/3/13; IgG1), MA30 (CD66c; clone 9A6; IgGI), MA41 (CD66b; clone BIRMA17c; IgGI), MA61 (CD66b; clone 80H3; IgGI), MA76 (CD66ae; clone 12-140-4; IgG1), MA79 (CD66b; clone B13.9; IgG1), MA81 (CD66b; clone G1OF5; IgGI), MA83 (CD66e; clone b7.8.5; IgG1), MA84 (CD66de; clone COL-1; IgG2a, MA86 (CD66acde; clone B6.2; IgG1) and MA91 (CD66e; clone T84.66; IgG1) are mouse mAbs which were obtained from the VIth Leukocyte Typing Workshop, Osaka, Japan. The isotype matched mouse IgG1 negative control mAb was purchased from Kakopatts (Copenhagen, Denmark) or Cappel (West Chester, PA). mAbs were purified by affinity purification and protein-A or G sepharose columns by standard methods.

Hybridoma 34B1, which produces monoclonal antibody 34B1, was deposited with the American Type Culture Collection (ATCC) on September 4, 2002, and assigned Accession Number ..........; Hybridoma 26H7, which produces monoclonal antibody 26H7, was deposited with the ATCC on September 4, 2002, and assigned Accession Number ..........; and Hybridoma 5F4, which produces monoclonal antibody 5F4, was deposited with the ATCC on September 4, 2002, and assigned Accession Number ..........

### EXAMPLE 2: Immunological and biological characteristics of monoclonal antibodies 34B1, 26H7 and 5F4

Some immunological and biological activities of monoclonal antibodies 34B1, 26H7 and 5F4 have been described in WO99/52552 and Morales et al., J. Immunol. 163 (1999) 1363-1370 which are hereby incorporated by reference. In particular, figures 1 to 5 and the corresponding examples in WO99/52552 show the immunological properties of antibodies 34B1, 26H7 and 5F4. Further assessment of specificity is published in Watt et al., Blood 98 (2001), 1469-1479, which describe that monoclonal antibody 5F4 binds to a domain within the N-Domain of C-CAM1 that is involved in homophilic interactions between the N-Domains of different C-CAM1 molecules.

Some biological activities of the monoclonal antibodies of the invention have also been described in WO99/52552 and Morales et al.; see supra. Thus, figures 7 and 8 and the corresponding examples in WO99/52552 disclose the inhibition of anti-CD3 directed and lymphokine activated killer activity of iIELs by anti-BGP antibodies and inhibition of human allo-mixed lymphocyte reaction by anti-BGP monoclonal antibodies, respectively. All the materials and methods disclosed in WO99/52552 and Morales at al., supra, for the characterisation of anti-BGP antibodies are incorporated herein by reference.
Thus, monoclonal antibodies 34B1, 26H7 and 5F4 produced by hybridomas ATCC , ATCC , and ATCC , inhibit T cell cytotoxicity and proliferation in an allogeneic mixed lymphocyte reaction in vitro. Furthermore, they inhibit cytokine production (interferon - gamma) as well. Mean radioactivity incorporated by cells is reported in Figs. 1-3 for various treatments including the antibodies 34B1 (Fig. 1), 26H7 (Fig. 2) and 5F4 (Fig. 3).

Human peripheral blood mononuclear cells (PBMC) were isolated as by standard methods. After washing three times, the cells were resuspended in complete medium containing human AB serum at 2×10⁶ cells/ml. The stimulator cells were irradiated with 3300 rads from a cesium source. Stimulator cells alone, responder cells alone or equal numbers of both were plated in 96 well U bottom plates at 4×10⁵ cells per well each in 200 µl. Increasing concentrations (1, 50 and 100 mg/ml) of the 34B1, 5F4, 26H7 or an isotype standard mouse anti-human IgGlk monoclonal antibody were added to the wells. Phytohemagglutinin-A (Murex, Dartford, U.K.) or PBS was added to control wells. Cells were incubated at 37°C and 5% CO₂ for 5 days. 18 h prior to harvest, 1 µCi of ³H-thymidine (New England Nuclear, Boston, MA) was added to each well. The cells were washed and harvested onto filters with a Tomtec Harvester 96 Mach II (Wallac, Gaithersburg, MD). After drying the filters, radioactivity was counted in the presence of scintillation fluid with a 2500 Betaplate liquid scintillation counter (Wallac). Each condition was performed in triplicate.

The complete disclosure of all patents, patent documents, and publications cited herein are incorporated by reference. The foregoing detailed description and examples have been given for clarity of understanding only. No unnecessary limitations are to be understood therefrom. The invention is not limited to the exact details shown and described, for variations obvious to one skilled in the art will be included within the invention claimed by the claims.

## Claims

1. An antibody that modulates T cell activity which is selected from the group consisting of monoclonal antibody 34B1 as produced by hybridoma 34B1, deposited with the American Type Culture Collection (ATCC) 10801 University Blvd. Manassas, VA 20110-2209 U.S.A. on September 4, 2002, and assigned Accession Number ..........; 26H7 as produced by hybridoma 26H7, deposited with the American Type Culture Collection (ATCC) on September 4, 2002, and assigned Accession Number .......... ; or 5F4 as produced by hybridoma 5F4, deposited with the American Type Culture Collection (ATCC) on September 4, 2002, and assigned Accession Number .............

2. A monoclonal antibody or antigen-binding fragment thereof that modulates T cell activity, wherein said antibody or antigen binding fragment thereof competes with an antibody of claim 1 for binding to the N-domain of BGP (C-CAM1).

3. The monoclonal antibody or antigen-binding fragment of claim 1 or 2 which is capable of inhibiting T cell proliferation in an allogenic mixed lymphocyte reaction (MLR) and/or suppressing the cytolytic activity of intestinal intraepithelial lymphocytes (iIELs).

4. A hybridoma that produces a monoclonal antibody of any one of claims 1 to 3.

5. A polynucleotide encoding at least a variable region of an immunoglobulin chain of the antibody of any one of claims 1 to 3.

6. A vector comprising the polynucleotide of claim 5, optionally in combination with a polynucleotide of claim 5 that encodes the variable region of the other immunoglobulin chain of said antibody.

7. A host cell comprising a polynucleotide of claim 5 or a vector of claim 6.

8. A method for preparing an antibody or a functional fragment or immunoglobulin chain(s) thereof, said method comprising
(a) culturing the hybridoma of claim 4 or the cell of claim 7; and
(b) isolating said antibody or functional fragment or immunoglobulin chain(s) thereof from the culture.

9. An antibody, an immunoglobulin chain thereof or a binding fragment thereof encoded by a polynucleotide of claim 5 or obtainable by the method of claim 8.

10. A pharmaceutical or diagnostic composition comprising the antibody of any one of claims 1 to 3 or 9, the polynucleotide of claim 5, the vector of claim 6 or the cell of claim 7.
